# EUROPÄISCHE PATENTSCHRIFT

(11) **EP 1 519 748 B1**
(45) Veröffentlichungstag und Bekanntmachung des Hinweises auf die Patenterteilung: **16.09.2009**
(21) Anmeldenummer: 03762386.5
(22) Anmeldetag: 30.05.2003
(51) Int. Cl.: A61K 39/44, A61K 9/50

(54) **MIKROPARTIKEL MIT CD28-SPEZIFISCHEN MONOKLONALEN ANTIKOERPERN**
MICROPARTICLE WITH CD28-SPECIFIC MONOCLONAL ANTIBODIES
MICROPARTICULES POURVUES D'ANTICORPS MONOCLONAUX SPECIFIQUES DE CD28

(30) Priorität: 04.07.2002 DE 10230223
(43) Veröffentlichungstag der Anmeldung: 06.04.2005
(73) Patentinhaber: TheraMAB GmbH, 97076 Würzburg (DE)
(72) Erfinder: HÜNIG, Thomas, 97286 Winterhausen (DE)
(74) Vertreter: Jungblut, Bernhard Jakob
(86) Internationale Anmeldenummer: PCT/DE2003/001825
(87) Internationale Veröffentlichungsnummer: WO 2004/004768

(56) Entgegenhaltungen:
- DE-A- 10 050 935
- US-A1- 2002 058 019
- BONYHADI M ET AL: "Xcellerate: An autologous T cell immunotherapy approach for treating B-cell lymphocytic leukemia (B-CLL)" BLOOD, W.B.SAUNDERS COMPAGNY, ORLANDO, FL, US, Bd. 96, Nr. 11 Part 1, 16. November 2000 (2000-11-16), Seite 837a XP000926673 ISSN: 0006-4971

## Beschreibung

### Gebiet der Erfindung

Die Erfindung betrifft Mikropartikel mit einer Trägerstruktur sowie mit einem an die Trägerstruktur gebundenen monoklonalen Antikörpern (mAb), Verwendungen solcher Antikörper sowie ein Verfahren zur Herstellung solcher Mikropartikel.

Hintergrund der Erfindung und Stand der Technik.

Es existieren verschiedene Erkrankungen bei Warmblütern, menschlich oder tierisch, wobei die Anzahl verschiedenartiger Blutzellen gegenüber dem Gesundzustand verringert oder unzureichend aktiv ist.

Eine erste solche Gruppe von Erkrankungen ist eine pathologisch erniedrigte T-Zellzahl, insbesondere CD4-T-Zellzahl gemeinsam, wie beispielsweise bei AIDS oder leukämischen Erkrankungen in Verfolg von Chemo- oder Strahlentherapie.

Eine zweite Gruppe von Erkrankungen, bei der die Zahl oder die Aktivität einer bestimmten Untergruppe von T-Zellen, die beim gesunden Menschen die immunologische Toleranz aufrecht erhalten, verringert oder in ihrer Funktion gestört ist, umfaßt autoimmun-inflammatorische Erkrankungen. Beispiele sind rheumatoide Arthritis, entzündliche Darmerkrankungen, Insulin-pflichtiger Diabetes, Multiple Sklerose sowie das Guaillan-Barre-Syndrom.

Eine dritte Gruppe von Erkrankungen wird Granulozytopenie (z.B. Neutropenie) bzw. Monozytopenie genannt und betrifft die Granulozyten. Als Ursachen für diese Erkrankung kommen in Frage: i) verminderte Granulozytopoese bzw. Monozytopoese (aplastische Störung) aufgrund von Knochenmarksschädigung, beispielsweise durch Chemikalien, wie Benzol, Medikamente, wie Zytostatika, Immunsupressiva, AZT und/oder Chloramphenicol (dosisabhängig, toxisch) oder wie Phenylbutazon, Goldverbindungen, selten Chloramphenicol (dosisunabhängig durch pharmakokinetische Reaktionen), Strahlen oder Autoantikörper gegen Stammzellen (bei manchen Fällen von Immunneutropenie), aufgrund von Knochenmarksinfiltration (Leukämien, Karzinome, maligne Lymphome) und/oder aufgrund von Osteomyelosklerose, ii) Reifungsstörungen der Granulozytopoese, beispielsweise durch kongenitale Reifungsstörungen der Myelopoese, Kostmann-Syndrom (Reifungsstop der Myelopoese im Stadium des Promyelozyten), zyklische Neutropenie, Myelodysplasie-Syndrom, Vitamin B12- oder Folsäuremangel mit ineffektiver Granulo-, Erythro- und/oder Thrombopoese. Übliche Therapien umfassen die Gabe von Wachstumsfaktoren der Granulozytopoese (beispielsweise G-CSF und GM-CSF).

Eine vierte Gruppe wird Thrombozytopenie genannt. Als Ursachen kommen in Frage: i) verminderte Thrombozytopoese im Knochenmark (aplastische Störung bzw. verminderte Megakaryozytenzahl im Knochenmark) aufgrund von Knochenmarksschädigung, beispielsweise durch Chemikalien, wie Benzol, Medikamente, wie Zytostatika und/oder Immunsupressiva, Strahlen Infektionen, wie z.B. HIV, oder Autoantikörper gegen Megakaryozyten (bei manchen Fällen von Immunthrombozytopenie), aufgrund von Knochenmarksinfiltration (Leukämien, Karzinome, maligne Lymphome) und/oder aufgrund von Osteomyelosklerose, ii) Reifungsstörungen der Megakaryozyten (Megakaryozyten im Knochenmark normal oder erhöht) mit ineffektiver Thrombo- Erythro- und/oder Granulopoese mit Megaloblasten, Riesenstäben u.a. aufgrund von Vitamin B12- oder Folsäuremangel. Übliche Therapien umfassen das Weglassen verdächtiger Medikamente, Thrombozytensubstitution (bei Bildungsstörungen im Knochenmark: Thrombopoetin) sowie MGDF (Stimulation der Proliferation und Ausreifung von Megakaryozyten.

Eine fünfte Gruppe bilden die aplastischen Anämien bzw. Knochenmarksversagen mit Aplasie/Hypoplaie des Knochenmarks und Panzytopenie (Stammzellenerkrankung). Eine angeborene aplastische Anämie ist beispielsweise die Fanconi-Anämie. Häufiger sind die erworbenen aplastischen Anämien, wie idiopatische aplastische Anämie (Ursache unbekannt) und sekundäre aplastische Anämien durch Medikamente, toxische Stoffe, ionisierende Strahlungen und Virusinfekte (s.o.). Supportive Therapieansätze umfassen die Substitution von Erythrozyten/Thrombozyten. Kausale Therapieansätze umfassen die Knochenmarkstransplantation oder Stammzellentransplantation, Immunsuppressive Therapien (z.B. ATG) und andere Therapiemaßnahmen, wie Gabe von Zytokinen (GM-CSF, G-CSF, MGDF, und/oder Thrombopoetin).

Schließlich kommt in Begleitung der akuten Leukämie oft Anämie, Thrombozytopenie und/oder Granulozytopenie, vor. Therapien umfassen die Substitution von Erythrozyten und Thrombozyten nach Bedarf bzw. die Anregung der Granulopoese durch G-CSF und/oder GM-CSF, die Chemotherapie und Knochenmarks- und/oder Stammzellentransplantation.

Den vorstehenden Erkrankungen der ersten und zweiten Gruppe ist gemeinsam, daß die betroffenen Blutzellen solche sind, die CD28 auf ihrer Oberfläche tragen. Der dritten bis sechsten Gruppe ist gemeinsam, daß die betroffenen Blutzellen solche sind, die demgegenüber kein CD28 auf ihrer Oberfläche tragen.

Zum Verständnis der Erfindung ist weiterhin folgender technologischer Hintergrund wichtig. Die Aktivierung ruhender T-Zellen zur Proliferation und funktionellen Differenzierung erfordert zunächst die Besetzung zweier Oberflächenstrukturen, sogenannter Rezeptoren: 1. des Antigenrezeptors, der von Zelle zu Zelle eine unterschiedliche Spezifität besitzt und für die Erkennung von Antigenen, z. B. viralen Spaltprodukten, notwendig ist; sowie des auf allen ruhenden T-Zellen mit Ausnahme einer Untergruppe der CD8 T-Zellen des Menschen gleichermaßen exprimierten CD28 Moleküls, welches natürlicherweise an Liganden auf der Oberfläche anderer Zellen des Immunsystems bindet. Man spricht von der Costimulation der antigenspezifischen Immunreaktion durch CD28. In Zellkultur können diese Vorgänge nachgestellt werden durch Besetzung des Antigenrezeptors sowie des CD28-Moleküls mit geeigneten mAbs. Im klassischen System der Costimulation führt weder die Besetzung des Antigenrezeptors noch die des CD28-Moleküls allein zur T-Zellproliferation, die Besetzung beider Rezeptoren ist jedoch effektiv. Diese Beobachtung wurde an T-Zellen des Menschen, der Maus und der Ratte gemacht.

Dagegen sind auch CD28-spezifische mAbs bekannt, die allein die T-Zellproliferation einleiten können. Eine solche superagonistische, d. h. von der Besetzung des Antigenrezeptors unabhängige Aktivierung ruhender T-Lymphozyten durch CD28-spezifische mAb, wurde in folgenden Systemen beobachtet: in der Literaturstelle Brinkmann et al., J. Immunology, 1996, 156: 4100-4106 wurde gezeigt, daß ein sehr kleiner Anteil (5 %) menschlicher T-Lymphozyten, die durch das Fehlen des Oberflächenmarkers CD45 RO der Gruppe der ruhenden T-Lymphozyten zugeordnet werden können, durch den normalerweise Costimulation erfordernden CD28-spezifischen mAb 9.3 bei Zusatz des Wachstumsfaktors Interleukin-2 (IL-2) ohne Besetzung des Antigenrezeptors aktiviert wird. In der Arbeit von Siefken et al., Cellular Immunology, 1997, 176: 59-65, wurde gezeigt, daß ein auf konventionellem Wege, d.h. durch Immunisierung von Mäusen mit menschlichen T-Zellen, hergestellter CD28-spezifischer mAb in Zellkultur eine Untergruppe menschlicher T-Zellen ohne Besetzung des Antigenrezeptors zur Proliferation aktivieren kann, wenn CD28 durch diesen mAb besetzt wird und die zellgebundenen mAb zusätzlich durch weitere Antikörper miteinander vernetzt werden. Den insofern bekannten Antikörpern ist gemeinsam, daß nur ein kleiner Anteil der T-Zellen aktivierbar ist.

In der Arbeit von Tacke et al., Eur. J. Immunol., 1997, 27:239-247 wurden zwei Arten von CD28-spezifischen monoklonalen Antikörpern mit unterschiedlichen funktionellen Eigenschaften beschrieben: costimulatorische mAb, die die Aktivierung ruhender T-Zellen nur bei gleichzeitiger Besetzung des Antigenrezeptors costimulieren; und superagonistische mAb, die ohne Besetzung des Antigenrezeptors T-Lymphozyten aller Klassen in vitro und im Versuchstier zur Proliferation aktivieren können. Beide insofern bekannte mAb rühren aus einer Immunisierung mit Zellen, auf denen Ratten-CD28 exprimiert ist und sind durch auf ihre jeweiligen beschriebenen Eigenschaften gerichtete unterschiedlichen Selektionen erhältlich. Schließlich ist aus der Literaturstelle WO 98/54225 ein weiterer human CD28 spezifischer superagonistischer mAb bekannt, nämlich CMY-2.

Überraschenderweise lassen sich gemäß der Literaturstelle DE-100 50 935 A1 mit superagonistischen CD28-spezifischen mAb bei in vivo Applikation aber auch Blutzellen stimulieren, welche kein CD28 tragen.

Die insofern bekannten superagonistischen mAb genügen in ihrer stimulatorischen Wirkung zwar allen Anforderungen, es wäre jedoch wünschenswert, weniger mAb für einen definierten stimulatorischen Effekt zu benötigen. Darüber hinaus ist die Stimulation von T-Lymphozyten durch superagonistische CD28-spezifische mAb bisher auf zwei Systeme beschränkt, die jeweils mit Nachteilen behaftet sind: Isolierte T-Lymphozyten können in Zellkultur oft nur dann durch solche mAb stimuliert werden, wenn zuvor die Kulturschalen mit Antikörpern beschichtet wurden, die mit den eingesetzten superagonistischen mAb reagieren und die sekundär quervernetzen. Bei Verwendung ungetrennter Präparationen peripherer Blutzellen, die auch T-Lymphozyten enthalten, sowie bei in vivo Applikation können die superagonistischen mAb auch als lösliche Substanz eingesetzt werden; dies liegt vermutlich daran, daß die sekundäre Quervernetzung durch sog. FC-Rezeptoren auf den nicht-T-Lymphozyten erfolgt, die mit dem konstanten Teil der superagonsitischen mAb, dem sog. Fc-Teil, reagieren. Diese Abhängigkeit von Fc-Rezeptoren ist u.U. wegen des variablen Anteils Fc-Rezeptor-positiver Zellen in Präparationen peripherer Blutzellen sowie wegen der durch verschiedene Fc-Rezeptor-Allele bedingten Variabilität der Antikörperbindungsfähigkeit problematisch. Darüber hinaus ist für eine wiederholte in vitro Stimulation von T-Lymphozyten durch lösliche superagonistische mAb der wiederholte Zusatz Fc-Rezeptor-exprimierender nicht-T-Zellen erforderlich. Für den Einsatz beim Menschen erfordert dies zusätzliche logistische und sicherheitstechnische Maßnahmen.

Bekannt ist es T-Zellen in Kultur dadurch zu vermehren, daß Kügelchen eingesetzt werden, an welchen sowohl CD28-spezifische costimulatorische mAb als auch TCR (CD3) spezifische mAb gebunden sind. Hierbei ist zunächst von Nachteil, daß zwei verschiedene Substanzen benötigt werden. Dies erfordert einen beachtlichen Aufwand bei der Herstellung unter GMP Bedingungen, welche für zur Therapie bestimmte Präparate gesetzlich vorgeschrieben sind. Weiterhin ist von Nachteil, daß jede dieser Substanzen in relativ hoher Konzentration eingesetzt werden muß, da aufgrund der gemeinsamen Immobilisierung auf einem Kügelchen nicht alle Moleküle für die notwendigerweise simultane Bindung Zielzellen allein schon aus sterischen Gründen zur Verfügung stehen.

### Technisches Problem der Erfindung.

Der Erfindung liegt daher das technische Problem zu Grunde, Mittel zur Verfügung zu stellen, mit welchen sich Blutzellen stimulieren lassen, wobei die Menge an benötigten mAbs niedriger ist als bei Einsatz der gleichen mAbs in Lösung, welche die Stimulation von der Anwesenheit bzw. der Qualität Fc-Rezeptor tragender nicht-T-Zellen unabhängig macht, und welche unter GMP Bedingungen einfacher herstellbar sind.

### Grundzüge der Erfindung und bevorzugte Ausführungsbeispiele.

Zur Lösung dieses technischen Problems lehrt die Erfindung Mikropartikel mit einer Trägerstruktur wobei an die Trägerstruktur ausschließlich CD28-spezifische superagonistische monoklonale Antikörper (mAb) oder eine Mimikryverbindung hierzu gebunden ist. Überraschenderweise wird für einen definierten stimulatorischen Effekt mittels solchermaßen immobilisierter mAb eine geringere Menge an mAbs benötigt bzw. wird bei gleicher Menge ein stärkerer stimulatorischer Effekt erreicht. Dies wird zudem erreicht, ohne daß ein TCR Signal benötigt wird. Die Bindung von TCR spezifischen Antikörpern oder anderen costimulatorischen Komponenten an der Trägerstruktur ist nicht erforderlich, wodurch auch sterische Probleme einer Costimulation durch immobilisierte Substanzen vermieden werden. Bei einer in vitro Stimulation von T-Zellen werden in der Zellkultur (neben dem Medium) nur zwei Komponenten benötigt, die T-Zellen und die erfindungsgemäßen Mikropartikel. Es entfällt die Notwendigkeit für "Feeder" Zellen oder die Beschichtung von Plastikoberflächen mit Antikörperpräparaten, die die mAbs quervernetzen.

In diesem Zusammenhang ist als weiterer Vorteil von besonderer Bedeutung, daß zur Herstellung erfindungsgemäßer Mikropartikel nur ein einziger Antikörper erforderlich ist, was die Herstellung unter GMP Bedingungen erheblich vereinfacht.

Im Einzelnen kann die Bindung der mAb an die Trägerstruktur auf die unterschiedlichste Weise erfolgen. So ist es möglich, daß die mAb direkt und über elektrostatische Kräfte, van der Waals Kräfte, hydrophobe Wechselwirkungen, vorzugsweise jedoch kovalent, an die Oberfläche der Trägerstruktur gebunden sind. Die mAb können aber auch indirekt über eine vorzugsweise kovalent mit der Oberfläche der Trägerstruktur verbundenen Spacerverbindung an die Oberfläche der Trägerstruktur gebunden sein.

Als Spacerverbindung kommen grundsätzlich alle organischen Moleküle in Frage, welche einerseits eine mit reaktiven Gruppen der Oberfläche der Trägerstruktur reaktive Gruppe aufweisen und andererseits in der Lage sind, mAbs zu binden. Dabei kann die Bindung der mAbs über elektrostatische Kräfte, van der Waals Kräfte, hydrophobe Wechselwirkungen oder kovalent erfolgen. Die Spacerverbindung kann insbesondere ausgewählt sein aus der Gruppe bestehend aus "organische Polymere, Peptide, Proteine, Immunglobuline, und Kombinationen solcher Substanzen". Die Spacerverbindung sollte physiologisch verträglich sein.

Hinsichtlich der Trägerstruktur ist deren Zusammensetzung unkritisch, solange eine Bindung der mAb oder einer Spacerverbindung an der Oberfläche einrichtbar ist. Es versteht sich, daß die Materialien der Trägerstruktur, jedenfalls soweit diese mit Zellen oder einer Lösung in Kontakt treten können, physiologisch verträglich sein sollten. Zweckmäßigerweise wird die Oberfläche der Trägerstruktur durch ein organisches Polymer gebildet, welches vorzugsweise ausgewählt ist aus der Gruppe bestehend aus "Polystyrol, Polyurethan, Polyester, Polyvinylpyridin, Poyvinylamin, Polyethylenimin, Chitosane, und Mischungen solcher Polymere". Das organische Polymer kann zum Zwecke der kovalenten Bindung von mAbs oder Spacerverbindungen reaktive Gruppen aufweist, welche vorzugsweise Glycidylether ist. Im Falle der Glycidylethergruppe erfolgt eine kovalente Bindung eines Proteins oder Peptids durch Reaktion primärer Amingruppen des Proteins oder Peptids mit dieser Gruppe.

Je nach eingesetztem Polymer kann es sich empfehlen, daß das organische Polymer oberflächenaktiviert ist durch Behandlung mit einem Aktivierungsreagenz, welches vorzugsweise p-Toluensulphonylchlorid ist.

Der Durchmesser der Trägerstruktur liegt zweckmäßigerweise im Bereich von 0,1 µm bis 100 µm, vorzugsweise im Bereich von 1 µm bis 20 µm, insbesondere im Bereich von 1 µm bis 10 µm. Die Partikelgröße kann beispielsweise mittels der Laserdiffraktometrie (Mastersizer x, Malvern Instruments, Herrsching) oder der Photonen Korrelations Spektroskopie (Zetasizer 4, Malvern Instruments, Herrsching) bestimmt werden. Die Oberfläche der Trägerstruktur kann das 1- bis 10-fache, vorzugsweise das 1- bis 4-fache, der geometrischen Oberfläche, angenommen als eine glatte Kugeloberfläche, betragen.

Geeignete Trägerstrukturen sind beispielsweise magnetische Beads, wie sie für die Zellseparation mittels Magneten verwendet werden, beispielsweise Dynabeads der Firma Dynal, oder auch SiO₂ Partikel. Weiterhin ist es möglich, Trägerstrukturen zu verwenden, welche vorzugsweise biologisch abbaubar sind, wodurch die Mikropartikel einem Patienten bedenkenlos verabreicht werden können. Geeignete Polymere für solche Trägerstrukturen sind beispielsweise in den Literaturstellen WO 01/05875, Kissel et al., Advanced Drug Delivery Reviews 54: 99-134 (2002), beschrieben. Bei ersteren handelt es sich im Kern um Polyethylenimine (PEI) und Derivate hiervon. Biologisch abbaubare Polymere können auch ladungsmodifizierte Polyester, Polyvinylpyridine oder Polyvinylamine sein.

Die Erfindung lehrt weiterhin die Verwendung von erfindungsgemäßen Mikropartikeln zur Stimulation von Blutzellen, insbesondere T-Lymphozyten, B-Lymphozyten, Granulozyten, Monozyten und/oder Thrombozyten. Dabei kann die Stimulation in vitro oder in vivo (letzteres insbesondere im Falle der CD28-negativen Zellen) erfolgen. Im Falle der T-Lymphozyten werden zumindest mehrere Untergruppen stimuliert. Daher ist auch die Verwendung zur Herstellung einer pharmazeutischen Zusammensetzung zur Behandlung von Erkrankungen mit reduzierten Blutzellzahlen, insbesondere reduzierten T-Lymphozytenzahlen, von immunpathologischen Erkrankungen (Beispiele s.u.) oder zur Potenzierung der Immunreaktion bei Impfungen umfaßt, wobei einem Patienten eine Blutprobe entnommen wird, wobei optional aus der Blutprobe die Blutzellen isoliert werden, wobei die Blutzellen in vitro unter Zugabe einer physiologisch wirksamen Dosis an Mikropartikeln kultiviert werden, und wobei die so erhaltenen Blutzellen optional galenisch zur Injektion oder Infusion hergerichtet werden. Ebenso ist die Verwendung erfindungsgemäßer Mikropartikel zur Herstellung einer pharmazeutischen Zusammensetzung zur Behandlung von Erkrankungen mit reduzierten Blutzellzahlen, von immunpathologischen Erkrankungen (beispielsweise rheumatoide Arthritis, entzündliche Darmerkrankungen, Insulin-pflichtiger Diabetes, Multiple Sklerose oder das Guillain-Barre-Syndrom) oder zur Potenzierung der Immunreaktion bei Impfungen umfaßt, wobei die Mikropartikel zur Injektion oder Infusion galenisch hergerichtet sind. Die galenische Herrichtung zur Injektion kann für die i.m., i.p. oder i.v. Injektion sein.

Die Erfindung betrifft schließlich ein Verfahren zur Herstellung von erfindungsgemäßen Mikropartikeln mit den folgenden Verfahrensschritten: a) es werden Mikropartikel mit einer aus einem oder mehreren verschiedenen organischen Polymeren gebildeten Oberfläche hergestellt, b) optional wird die Oberfläche aktiviert, c) die so erhaltenen Mikropartikel werden mit einer Lösung enthaltend CD28-spezifische superagonistische mAb inkubiert, wobei die mAb vorzugsweise kovalent an die Oberfläche gebunden werden, oder c') die so erhaltenen Mikropartikel werden zunächst mit einer Lösung enthaltend eine Spacerverbindung inkubiert, wobei die Spacerverbindung vorzugsweise kovalent an die Oberfläche gebunden wird, optional erfolgt danach eine Waschverfahrensstufe, und anschließend werden die Mikropartikel mit der gebundenen Spacerverbindung mit einer Lösung enthaltend CD28-spezifische superagonistische mAb inkubiert, wobei die mAb an die Spacerverbindung kovalent oder nicht-kovalent gebunden werden, und e) die so erhaltenen CD28-spezifische superagonistische mAb tragenden Mikropartikel werden aus der Lösung abgetrennt und optional einer Waschverfahrensstufe unterworfen.

Die Erfindung betrifft schließlich auch Verfahren zur Prophylaxe und/oder Behandlung der beschriebenen Erkrankungen, wobei einem Patienten entweder die erfindungsgemäßen Mikropartikel, galenisch geeignet hergerichtet, verabreicht werden, oder wobei vorzugsweise autologe Zellen in vitro mittels erfindungsgemäßer Mikropartikel stimuliert und nach der Stimulation ohne oder mit den Mikropartikeln, galenisch geeignet hergerichtet, implantiert werden.

Erläuterungen zu den Mikropartikeln gelten sinngemäß auch für die erfindungsgemäßen Verwendungen und Verfahren.

### Definitionen.

Die Aminosäuresequenz von Human CD28 ist unter der Accession No. NM_006139 bekannt.

Superagonistische CD28 spezifische mAb sind beispielsweise aus Hybridomzellen erhältlich, die unter den Hinterlegungsnummern DSM ACC2530 und DSM ACC2531 hinterlegt sind. Humanisierte mAb entsprechend DSM ACC2530 weisen Sequenzen der leichten Kette sowie der schweren Kette gemäß den SEQ.-ID 1 und 2 auf.

Mit Stimulierung bzw. Aktivierung von Blutzellen, insbesondere T-Lymphozyten, ist die Vermehrung der Stoffwechselaktivität, Vergrößerung des Zellvolumens, Synthese immunologisch wichtiger Moleküle und Eintritt in die Zellteilung (Proliferation) auf einen äußeren Reiz hin bezeichnet.

Mehrere Untergruppen der T-Zellen meint zumindest die Untergruppen der CD4 und CD8 T-Zellen. Zu den CD4 T-Zellen zählen neben den "normalen" CD25-negativen T-Zellen auch die CD4⁺CD25⁺-Zellen, welche auch als regulatorische T-Zellen bekannt sind. Nach Befunden in Tiermodellen geht der derzeitige Stand der Technik davon aus, daß letztere bei zahlreichen autoimmunen und inflammatorischen Erkrankungen, wie beispielsweise der Multiplen Sklerose, Guillian-Barré-Syndrom, demyelinisierender Polyneuropathie, etc, eine Rolle spielen. Ein nützlicher Marker für regulatorische T-Zellen ist die starke Expression von CTLA-4 (CD152), das von nicht-regulatorischen T-Zellen nur nach Aktivierung und dann auch nur in geringem Maße exprimiert wird.

Der Begriff der mAb umfaßt neben Strukturen des üblichen Fab/Fc Aufbaus auch Strukturen, die ausschließlich aus dem Fab-Fragment bestehen. Es ist auch möglich, lediglich die variable Region zu nutzen, wobei das Fragment der schweren Ketten mit dem Fragment der leichten Kette auf geeignete Weise, beispielsweise auch mittels synthetischer Brückenmoleküle, so verbunden ist, daß die Bindungsregionen der Ketten die Antikörperbindungsstelle bilden. Der Begriff der Antikörper umfaßt auch (vollständige) chimäre sowie humanisierte Antikörper.

Der Begriff der mAb umfaßt weiterhin zu den eigentlichen mAbs analoge Peptide oder Proteine. Ein analoges Peptid oder Protein ist ein Peptid oder Protein, dessen Aminosäuresequenz von jener des Peptids oder Proteins, zu welchem es analog ist, abweicht, jedoch einen definierten Bindungspartner des eigentlichen mAb mit zumindest gleicher Affinität bindet. Abweichungen in der Sequenz können Deletionen, Substitutionen, Insertionen und Elongationen sein. Ein analoges Peptid oder Protein wird in der Regel eine dem Peptid oder Protein sehr ähnliche Tertiär(teil)struktur und/oder Exposition im Rahmen eines (Zelloberflächen-) Proteins aufweisen und braucht ansonsten nur in dem dem unmittelbaren Bindungsbereich des Peptids oder Proteins analogen Bereich eine Bindungsstelle für den definierten Bindungspartner aufzuweisen bzw. diese bilden.

Eine Mimikryverbindung eines mAb ist eine natürliche oder synthetische chemische Struktur, die sich in einem Bindungsassay wie ein definierter mAb verhält, welchen die Mimikryverbindung mimikriert.

Superagonistische Modulation der Proliferation von Blutzellen, insbesondere T-Zellen meint, daß keine Costimulation, i.e. kein weiteres Bindungsereignis neben einer (initialen) Bindung eines mAb oder einer Mimikryverbindung an CD28 zur Stimulation oder Inhibierung der Proliferation erforderlich ist.

Der Begriff der Behandlung umfaßt auch den Begriff der Prophylaxe.

Im Folgenden wird die Erfindung anhand von lediglich Ausführungsformen darstellenden Beispielen näher erläutert.

### Beispiel 1: direkte Koppelung von mAb an magnetische Beads.

Eingesetzt werden oberflächenaktivierte magnetische Beads des Firma Dynal (Dynabeads). Die Oberfläche der Beads ist hydrophob und trägt Glycidylethergruppen. Eine kovalente Bindung von Antikörpern erfolgt durch Reaktion primärer Amingruppen der Antikörper mit den Glycidylgruppen. Die Beads sind einheitlich geformte, superparamagnetische Kügelchen mit einem Kern aus γFe₂O₃ und Fe₃O₄, welcher mit einer Polystyrolhülle überzogen ist. Der mittlere Durchmesser beträgt 4,5 µm. Die Dichte beträgt ca. 1,5 g/cm³. Die Oberfläche beträgt 1 bis 4 m²/g Beads. Die geometrische Oberfläche beträgt 6x10⁻⁴m²/10⁷ Dynabeads (ca. 0,9 m²/g). Die magnetischen Eigenschaften sind im Rahmen der Erfindung irrelevant; helfen allerdings bei der Handhabung im Rahmen der Präparation.

Die gewünschte Anzahl Beads wird der gelieferten Suspension nach gründlicher Mischung der Suspension (Vortex, 5 min.) entnommen. Das Gefäß mit den Beads wird in eine magnetische Halterung eingesetzt (30-60 s), wodurch die Beads pelletiert werden. Der Überstand wird abgenommen und Kopplungspuffer, in welchem die Bindung der Antikörper an die Beads erfolgen soll (0,1 M H₃BO₄, pH 8,5, eingestellt mit NaOH), wird zugesetzt. Die Beads werden gründlich resuspendiert (Vortex, mindestens 2 min.) und auf diese Weise gewaschen. Dieser Waschschritt wird insgesamt dreimal durchgeführt.

Für die Kopplung des Antikörpers werden die Beads in frischem Kopplungspuffer zusammen mit dem CD28 spezifischen superagonistischen Antikörper (huIgG-5.11, voll humanisierter mAb des IgG 4 Isotyps, gentechnologisch abgeleitet von einem mAb aus DSM ACC2530) sorgfältig resuspendiert (Vortex, 30-60 s). Die Konzentration der Beads beträgt 4x10⁸/ml. Je 10⁷ Beads werden 5 µg gereinigter mAb eingesetzt. Das Inkubationsgefäß mit dem Ansatz wird an einem Rotator befestigt, durch langsame Rotation des Inkubationsgefäßes wird die Sedimentation der Beads und damit eine Entmischung des Reaktionsansatzes verhindert. Die Inkubation erfolgt 20-24 h zwischen 4°C und 37°C, beispielsweise 20°C.

Nach der Inkubation werden die Beads mit Hilfe der magnetischen Halterung pelletiert, der Überstand wird abgenommen und für eine Proteingehaltmessung aufbewahrt. Die Beads werden in Waschpuffer (PBS ohne Ca²⁺ und Mg²⁺, 0,1% BSA) resuspendiert. Die Suspension wird auf dem Rotator bei langsamer Drehung 5 min. bei 4°C gewaschen. Dieser Waschschritt wird insgesamt dreimal mit jeweils frischem Waschpuffer durchgeführt. Nach Abschluß der Waschschritte werden die Beads in Waschpuffer resuspendiert und sind verwendungsfähig. Die Lagerung erfolgt bei 4°C.

Eine Messung des Proteingehaltes der Überstandes aus der Koppelung ergibt einen Anteil von ca. 50% der eingesetzten Menge, so daß maximal 50% der eingesetzten Menge als gekoppelt angenommen werden können. Hieraus errechnet sich eine Mindestanzahl Beads von 4x10⁶/µg gekoppelter mAb, oder 2,5 µg mAb/10⁷ Beads.

### Beispiel 2: indirekte Koppelung von mAb an magnetische Beads.

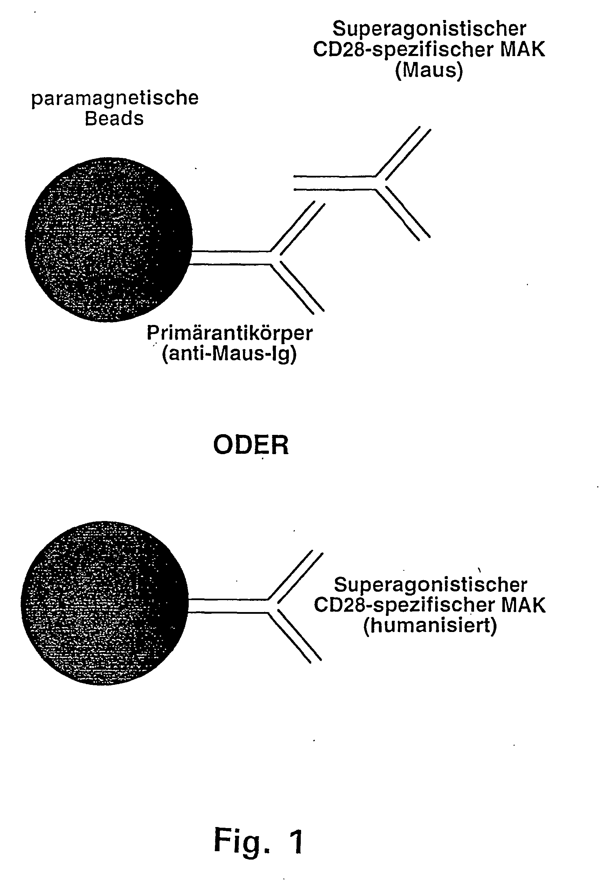

Es wird grundsätzlich analog dem Beispiel 1 vorgegangen, nur daß anti-Maus Ig Dynabeads verwendet werden. Hierbei handelt es sich um Beads, an deren Oberfläche Schaf-anti-Maus Immunglobulin kovalent gekoppelt ist. Als Kopplungspuffer wird Phosphat-gepufferte physiologische Kochsalzlösung (PBS)eingesetzt. Als mAb wurden Maus anti-human CD28 spezifische superagonistische mAb eingesetzt, welche erhältlich sind aus DSM ACC 2530. Deren Spezifität entspricht vollumfänglich jener der in Beispiel 1 eingesetzten mAb, da Übereinstimmung in den Antigen-Bindungsbereichen besteht. An das Immunglobulin bindet der eingesetzte mAb nicht-kovalent gemäß der Figur 1.

### Beispiel 3: in vitro Stimulation in einem PBMC System.

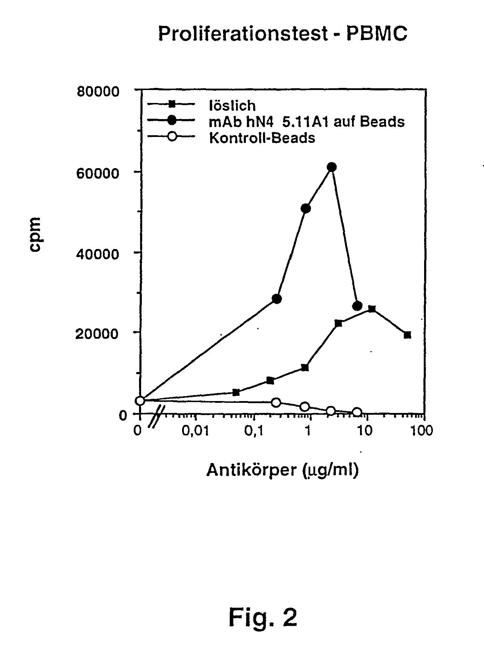

Pro Napf einer 96 Lochplatte wurden 2x10⁵ PBMC (periphere Blut mononukleäre Zellen, i.e. T-Lymphozyten, B-Lymphozyten und Monozyten) in 0,2 ml RPMI 1640 Kulturmedium mit 10% autologem Serum kultiviert. Von Tag 2 auf Tag 3 wurde ein 16-stündiger Puls mit 1 µCi ³H-Thymidin durchgeführt, worauf das in die DNA eingebaute Thymidin als Nachweis der Proliferation gemessen wurde. Die Ergebnisse der Figur 2 vergleichen die Fähigkeit des humanisierten superagonistischen mAb hN4 5.11A1, ungetrennte menschliche PBMC (also eine Mischung aus T-Lymphozyten und Fc Rezeptor tragenden nicht-T-Lymphozyten) in löslicher gegenüber bead-gekoppelter Form zu stimulieren. Dabei ist auf der Abszisse die jeweils tatsächlich in das System eingebrachte Antikörpermenge (in löslicher Form oder gebunden an Beads) angegeben. Die Ordinate zeigt die eingebaute Radioaktivität als Maß der Proliferation. Dieses repräsentative Experiment zeigt eindeutig, daß der an Beads gekoppelte Antikörper erstens im Optimum eine weitaus stärkere Proliferation induziert als der nur löslich zugegebene, und zweitens, daß die spezifische Aktivität des Antikörpers im Sinne der Proliferationsinduktion bei Bindung an Beads etwa 10-fach höher liegt als bei Zugabe des isolierten Antikörpers.

### Beispiel 4: in vitro Stimulation von isolierten T-Lymphozyten.

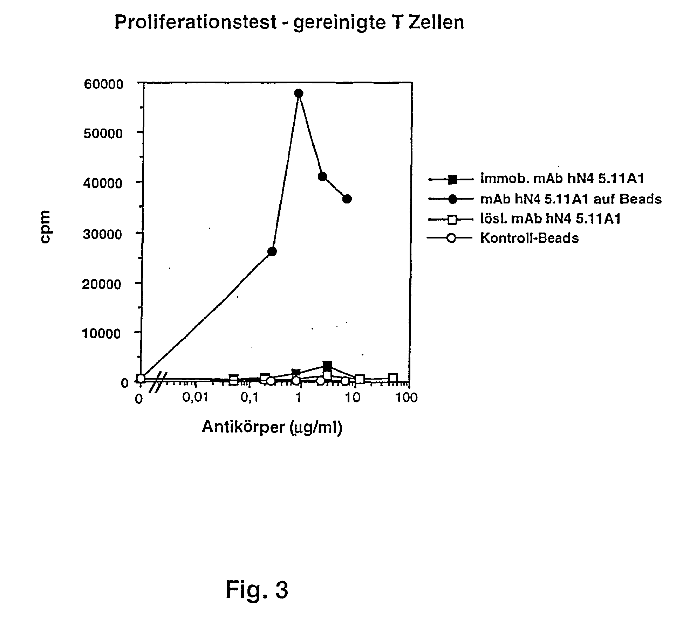

Unter ansonsten gleichen Kultivierungs- und Messbedingungen wie in Beispiel 3 wurden pro Napf 2x10⁵ gereinigte T-Lymphozyten eingesetzt (gewonnen mittels Nylonwollfiltration). In Figur 3 ist ein Vergleich bei Einsatz von Mikropartikeln aus Beispiel 1 mit einem Einsatz der in Beispiel 1 eingesetzten, jedoch nicht mit mAb gekoppelten Mikropartikeln dargestellt. Zusätzlich wird der stimulierende Effekt mit lösliche zugesetzten mAbs in An- und Abwesenheit eines an die Plastikoberfläche gekoppelten Sekundärantikörpers (Esel anti-human Ig) verglichen. Man erkennt, dass unkonjugierte Mikropartikel keine Proliferation auslösen, während die mAb tragenden Mikropartikel eine sehr starke Aktivität zeigen. Diese ist um ein Vielfaches höher als die Aktivität, welche durch lösliche mAb in An- oder Abwesenheit des immobiliiserten Sekundärantikörpers erreicht wird. Die im Vergleich zu ungetrennten PBMC (Figur 2) sehr geringe Stimulation durch löslich zugesetzten Antikörper ist erwartet, da Zellen mit Rezeptoren für das Fc Stück des Antikörpers (FcR) fehlen. Die Anwesenheit des immobilisierten Sekundärreagenzes führt zu einer leichten Steigerung der Reaktivität auf löslich zugesetzten Antikörper. Die Beads übernehmen die Funktion Fc Rezeptor positiver Zellen jedoch um ein Vielfaches effizienter.

### Beispiel 5: Stimulierbare T-Zell Untergruppen.

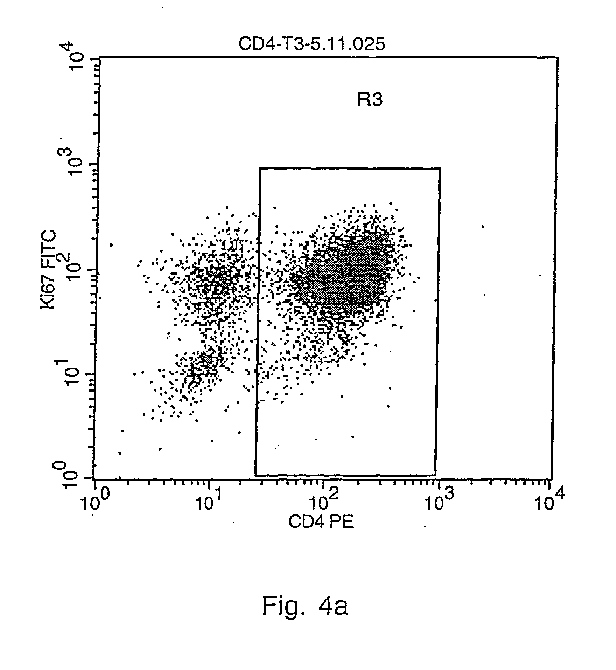

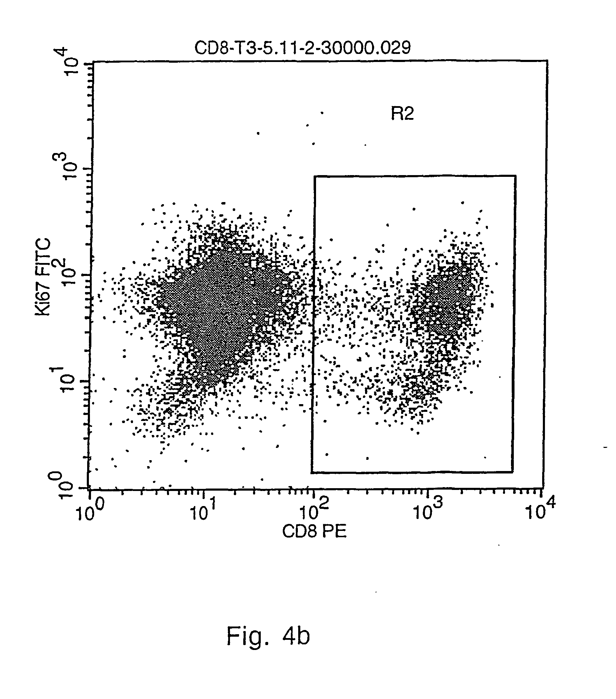

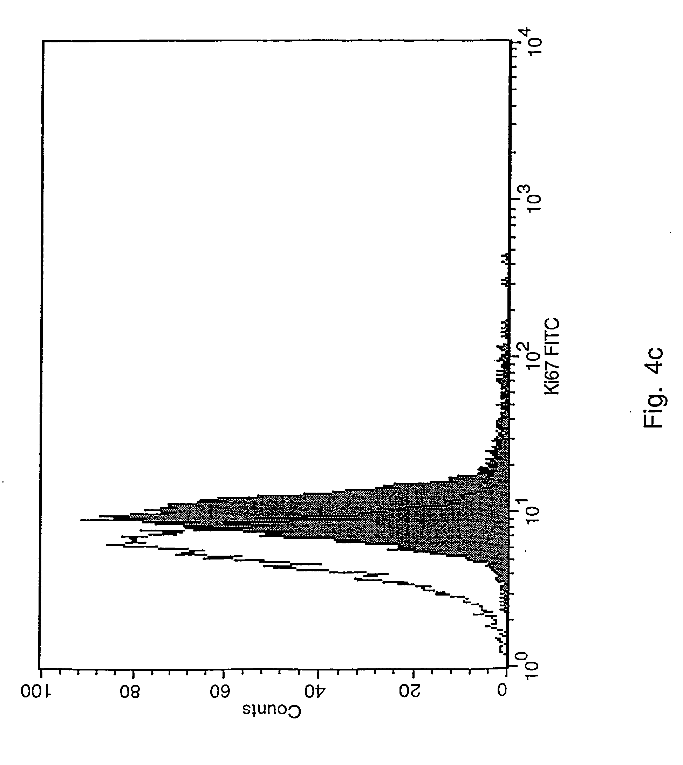

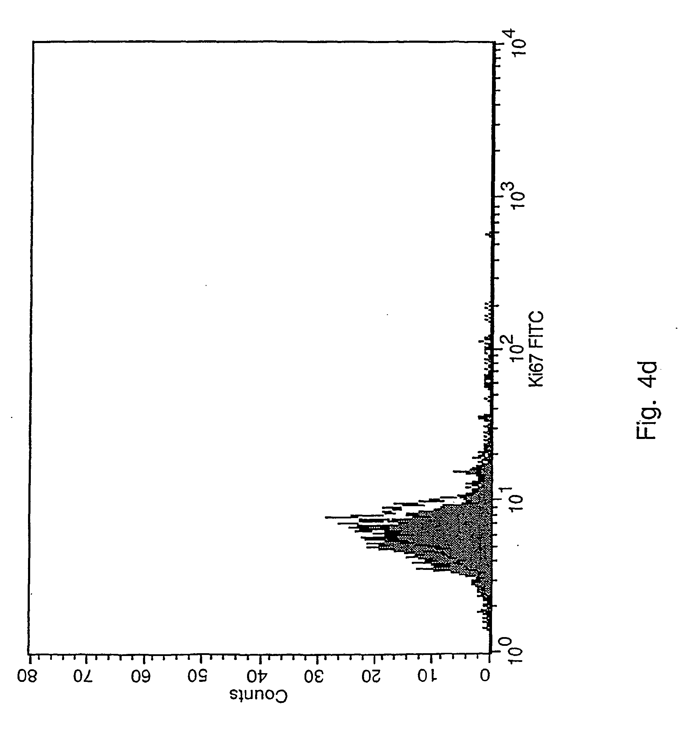

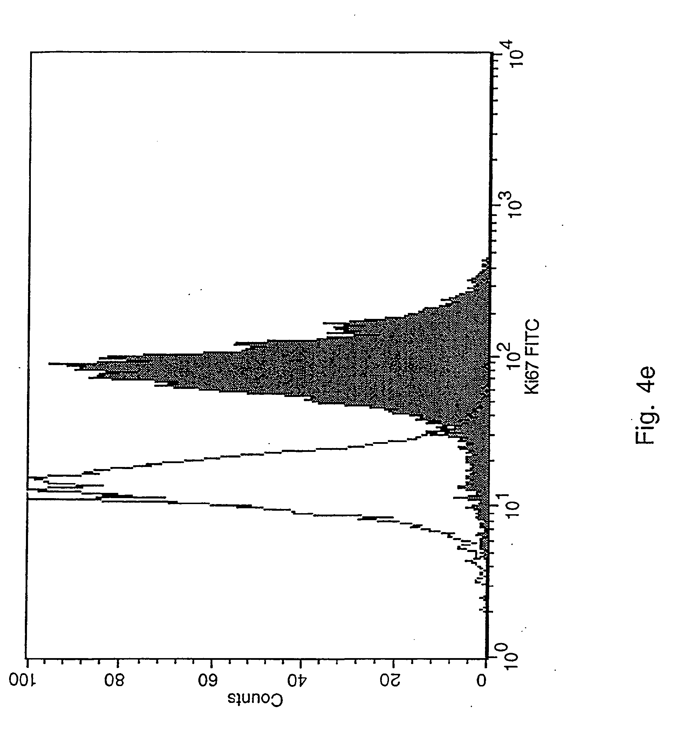

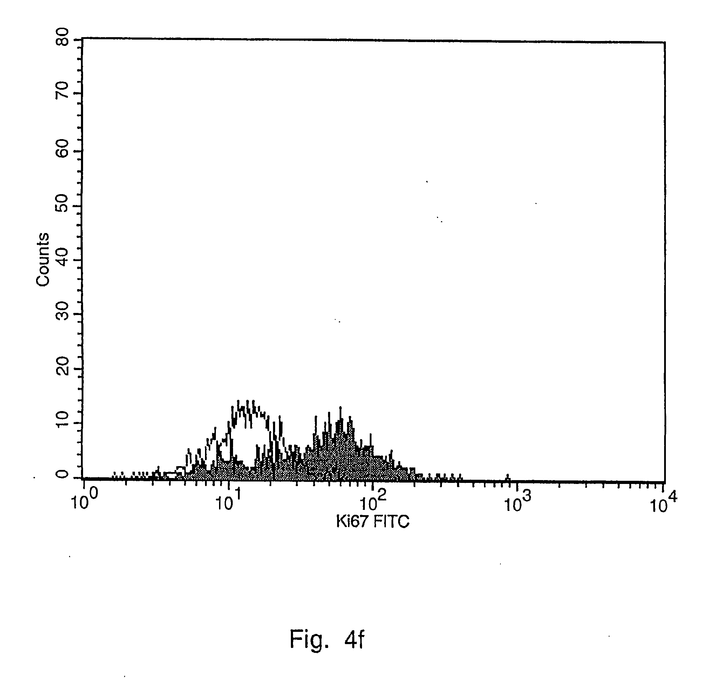

Gereinigte T-Lymphozyten wurden entsprechend dem Beispiel 4 kultiviert, wobei Mikropartikel gemäß Beispiel 2 eingesetzt wurden. Nach 3-tägiger Kultur wurden die Zellen im FACS auf die Expression des nukleären Proliferationsmarkers Ki67 nach intrazellulärer Färbung untersucht. Dabei wurden selektiv die CD4 bzw. die CD8 T-Zellen durch elektronisches Gating nach gleichzeitiger Anfärbung mit einem CD4 oder CD8 spezifischen Antikörper analysiert. Die gesetzten Fenster sind in den Figuren 4a (CD4⁺-Zellen) und 4b (CD8⁺-Zellen) dargestellt. PE steht für den Fluoreszenzfarbstoff Phycoerythrin. In die Histogramme der Figuren 4c bis 4f gingen jeweils nur die Zellen aus dem jeweils eingekastelten Bereich ein (Figuren 4c und 4e: CD4⁺-Zellen; Figuren 4d und 4f: CD8⁺-Zellen). In den Histogrammen erfolgte eine Kontrollfärbung mit einem irrelevanten Antikörper (offene Kurven) sowie die Ki67 Färbung. FITC steht für den Fluoreszenzfarbstoff Fluorescein Isothiozyanat. Eine vergleichende Betrachtung der Figuren 4c und 4d einerseits sowie der Figuren 4e und 4f andererseits zeigt, daß ohne Stimulation Kontroll- und Ki67 Histogramme sich kaum voneinander absetzen. Dagegen sind bei Stimulation mit den erfindungsgemäßen Mikropartikeln praktisch alle CD4 T-Zellen und die Mehrheit der CD8 T-Zellen Ki67-positiv und proliferieren folglich. Die Histogramme der Figuren 4d und 4f sind flacher als jene der Figuren 4c und 4e, weil weniger CD8 als CD4 T-Zellen vorhanden sind.

### Beispiel 6: Stimulation regulatorischer T-Zellen.

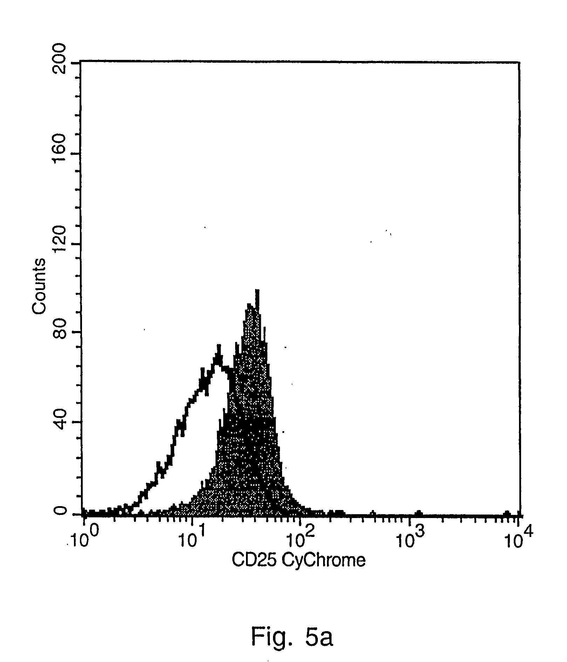

Es wurden menschliche CD4 T-Zellen durch Nylonwoll-Filtration und immun-magnetische Depletion aller anderen PBMC-Populationen gereinigt. Hierauf wurden die erhaltenen CD4 T-Zellen in CD25 positive und CD25 negative Fraktionen aufgetrennt mittels der magnetischen Zellsortierung (System MACS, Miltenyi Biotech, Bergisch Gladbach, Deutschland). In Figur 5a ist die Histogrammform die CD25 Expression der beiden gereinigten Populationen von CD4 T-Zellen dargestellt. Die CD25 Expression wird durch die Rechtsverschiebung der ausgefüllten Verteilungskurve der Fluoreszenz (CyChrome-gekoppelte CD25 spezifische mAb) gegenüber der leeren Kurve für CD25-negative Zellen ersichtlich.

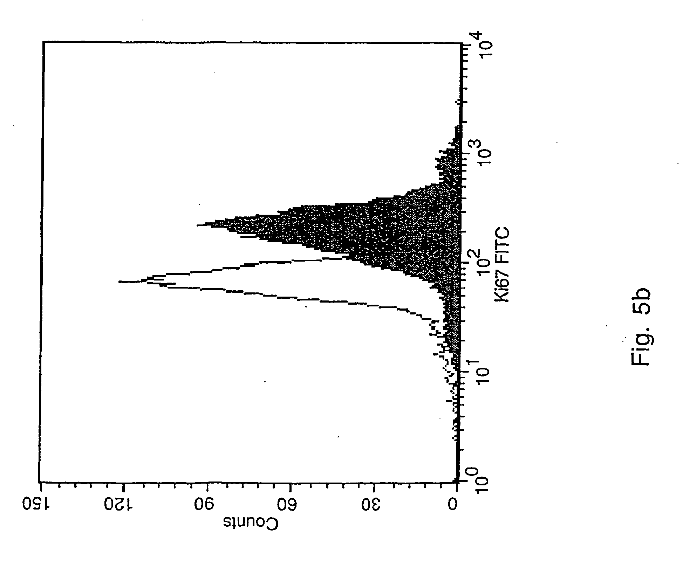

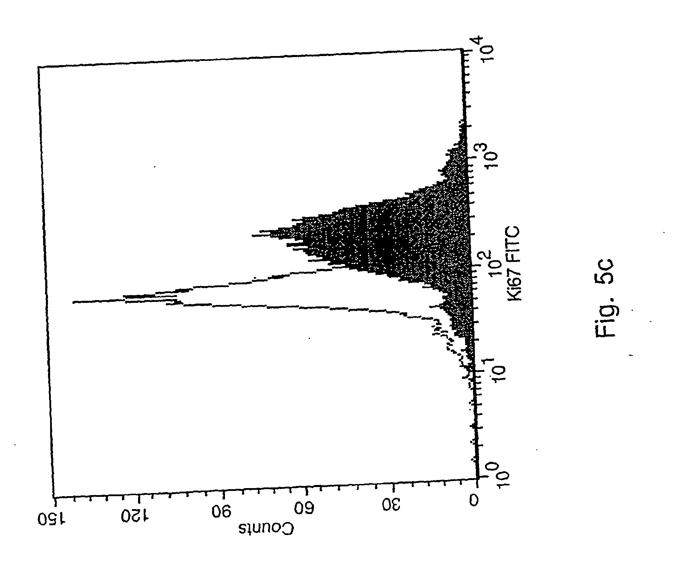

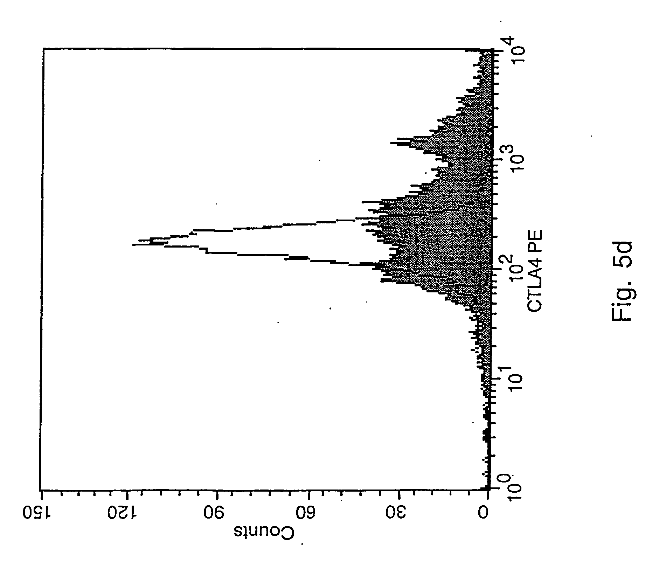

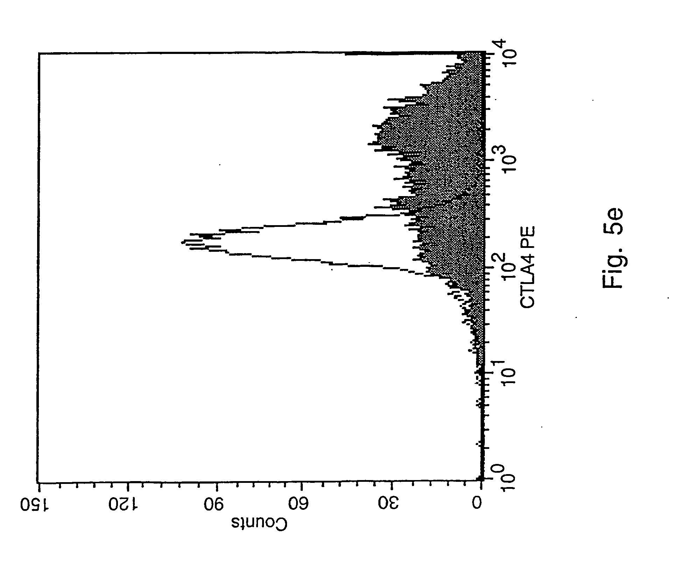

Die wie vorstehend erhaltenen CD4 T-Zellen wurden 3 Tage mit Mikropartikeln gemäß Beispiel 2 stimuliert. Dann wurde durch intrazelluläre Färbung die Expression von Ki67 (Proliferation) und CTLA-4 (starke Expression ist indikativ für regulatorische T-Zellen) analysiert. Die Figuren 5b und 5d zeigen die Ergebnisse für CD25-negative T-Zellen, wobei die offenen Kurven wiederum mit einem irrelevanten mAb zu Kontrollzwecken erhalten wurden. Figur 5b belegt, daß die CD25⁻ Zellen proliferieren. Nur ein kleiner Teil der CD25⁻ Zellen exprimieren CTLA-4, wie aus der Figur 5d ersichtlich. Figur 5c zeigt, daß auch die CD25⁺ Zellen proliferieren. Figur 5e belegt, daß die Mehrheit der CD25⁺ Zellen stark CTLA-4 exprimiert. Im Ergebnis ist gezeigt, daß nicht nur CD25⁻ T-Zellen, sondern insbesondere auch CD25⁺ T-Zellen mit den erfindungsgemäßen Mikropartikeln stimulierbar sind.

## Patentansprüche

1. Mikropartikel mit einer Trägerstruktur wobei an die Trägerstruktur ausschließlich CD28-spezifische superagonistischen monoklonalen Antikörper (mAb) oder eine Mimikryverbindung hierzu gebunden ist.

2. Mikropartikel nach Anspruch 1, wobei die mAb direkt und vorzugsweise kovalent an die Oberfläche der Trägerstruktur gebunden sind.

3. Mikropartikel nach Anspruch 1, wobei die mAb indirekt über eine vorzugsweise kovalent mit der Oberfläche der Trägerstruktur verbundene Spacerverbindung an die Oberfläche der Trägerstruktur gebunden sind.

4. Mikropartikel nach Anspruch 3, wobei die Spacerverbindung ausgewählt ist aus der Gruppe bestehend aus "organische Polymere, Peptide, Proteine, und Kombinationen solcher Substanzen".

5. Mikropartikel nach einem der Ansprüche 1 bis 4, wobei die Oberfläche der Trägerstruktur durch ein organisches Polymer gebildet wird, welches vorzugsweise ausgewählt ist aus der Gruppe bestehend aus "Polystyrol, Polyurethan, Polyester, Polyvinylpyridin, Polyvinylamin, Polyethylenimin, Chitosane, und Mischungen solcher Polymere".

6. Mikropartikel nach Anspruch 5, wobei das organische Polymer reaktive Gruppen aufweist, welche beispielsweise Glycidylether ist.

7. Mikropartikel nach Anspruch 5 oder 6, wobei das organische Polymer oberflächenaktiviert ist durch Behandlung mit einem Aktivierungsreagenz, welches vorzugsweise p-Toluensulphonylchlorid ist.

8. Mikropartikel nach einem der Ansprüche 1 bis 7, wobei der Durchmesser der Trägerstruktur im Bereich von 0,1 µm bis 100 µm, vorzugsweise im Bereich von 1 µm bis 20 µm, insbesondere im Bereich von 1 µm bis 10 µm, liegt.

9. Mikropartikel nach einem der Ansprüche 1 bis 8, wobei die Oberfläche der Trägerstruktur (gemessen mittels der BET-Methode) das 1- bis 10-fache, vorzugsweise das 1-bis 4-fache, der geometrischen Oberfläche, angenommen als eine glatte Kugeloberfläche, beträgt.

10. Verwendung von Mikropartikeln nach einem der Ansprüche 1 bis 9 zur Herstellung von Mitteln zur Stimulation von Blutzellen, insbesondere von T-Lymphozyten, B-Lymphozyten, Granulozyten, Monozyten und/oder Thrombozyten.

11. Verwendung nach Anspruch 10 zur Herstellung einer pharmazeutischen Zusammensetzung zur Behandlung von Erkrankungen mit reduzierten Blutzellzahlen, insbesondere reduzierten T-Lymphozytenzahlen, oder von immunpathologischen Erkrankungen oder zur Potenzierung der Immunreaktion bei Impfungen, wobei einem Patienten eine Blutprobe entnommen wird, wobei optional aus der Blutprobe die Blutzellen isoliert werden, wobei die Blutzellen in vitro unter Zugabe einer physiologisch wirksamen Dosis an Mikropartikeln kultiviert werden, und wobei die so erhaltenen Blutzellen optional galenisch zur Injektion oder Infusion hergerichtet werden.

12. Verwendung nach Anspruch 10 zur Herstellung einer pharmazeutischen Zusammensetzung zur Behandlung von Erkrankungen mit reduzierten Blutzellzahlen oder von immunpathologischen Erkrankungen oder zur Potenzierung der Immunreaktion bei Impfungen, wobei die Mikropartikel galenisch vorzugsweise zur Injektion oder Infusion hergerichtet werden.

13. Verfahren zur Herstellung von Mikropartikeln nach einem der Ansprüche 1 bis 9, mit den folgenden Verfahrensschritten:
a) es werden Mikropartikel mit einer aus einem oder mehreren verschiedenen organischen Polymeren gebildeten Oberfläche hergestellt,
b) optional wird die Oberfläche aktiviert,
c) die so erhaltenen Mikropartikel werden mit einer Lösung enthaltend CD28-spezifische superagonistische mAb inkubiert, wobei die mAb vorzugsweise kovalent an die Oberfläche gebunden werden, oder
c') die so erhaltenen Mikropartikel werden zunächst mit einer Lösung enthaltend eine Spacerverbindung inkubiert, wobei die Spacerverbindung vorzugsweise kovalent an die Oberfläche gebunden wird, optional erfolgt danach eine Waschverfahrensstufe, und anschließend werden die Mikropartikel mit der gebundenen Spacerverbindung mit einer Lösung enthaltend CD28-spezifische superagonistische mAb inkubiert, wobei die mAb an die Spacerverbindung kovalent oder nicht-kovalent gebunden werden, und
e) die so erhaltenen CD28-spezifische superagonistische mAb tragenden Mikropartikel werden aus der Lösung abgetrennt und optional einer Waschverfahrensstufe unterworfen.

## Claims

1. Microparticle with support structure, wherein exclusively CD28-specific superagonistic monoclonal antibodies (mAb) or a mimicry-compound hereof are bound to the support structure.

2. Microparticle according to claim 1, wherein the mAb are bound directly and preferably covalently to the surface of the support structure.

3. Microparticle according to claim 1, wherein the mAb are bound indirectly to the surface of the support structure via a spacer-compound being preferably covalently bound to the surface of the support structure.

4. Microparticle according to claim 3, wherein the spacer-compound is selected from the group consisting of "organic polymers, peptides, proteins, and combinations of such compounds".

5. Microparticle according to one of the claims 1 to 4, wherein the surface of the support structure is formed by an organic polymer, which is preferably selected from the group consisting of "polystyrol, polyurethan, polyester, polyvinylpyridine, polyvinylamine, polyethylenimine, chitosane, and mixtures of such polymers".

6. Microparticle according to claim 5, wherein the organic polymer comprises reactive groups, which for example are glycidylether.

7. Microparticle according to claim 5 or 6, wherein the organic polymer is surface-activated by treatment with an activation agent, which preferably is p-toluensulphonylchloride.

8. Microparticle according to one of the claims 1 to 7, wherein the diameter of the support structure is in the range from 0,1 µm to 100 µm, preferably in the range from 1 µm to 20 µm, in particular in the range of 1 µm to 10 µm.

9. Microparticle according to one of the claims 1 to 8, wherein the surface of the support structure (measured using the BET-method) is 1- to 10-fold, preferably 1- to 4-fold, of the geometric surface, assumed as a smooth spheric surface.

10. Use of microparticles according to one of the claims 1 to 9 for the preparation of means for stimulation of blood cells, in particular of T-lymphocytes, B-lymphocytes, granulocytes, monocytes and/or thrombocytes.

11. Use according to claim 10 for manufacturing a pharmaceutical composition for the treatment of diseases with reduced numbers of blood cells, in particular reduced numbers of T-lymphocytes or of immunpathological diseases or for enhancement of immunoreactions after immunization, wherein a blood specimen is taken from a patient, wherein the blood cells are optionally isolated from the blood specimen, wherein the blood cells are cultivated in vitro under addition of a physiological effective dosage of microparticles, and wherein the in this way obtained blood cells are galenically prepared, optionally for injection or infusion.

12. Use according to claim 10 for manufacturing a pharmaceutical composition for the treatment of diseases with reduced numbers of blood cells or for immunpathological diseases or enhancement of the immunoreactions upon immunization, wherein microparticles are galenically prepared preferably for injection or infusion.

13. Method for the production of microparticles according to one of the claims 1 to 9 with the following steps:
a) Microparticles with a surface made of one or several different organic polymers are prepared,
b) the surface is optionally activated,
c) the thus obtained microparticles are incubated with a solution comprising CD28-specific superagonistic mAb, wherein the mAb are bound to the surface, preferably covalently, or
c') the thus obtained microparticles are first incubated with a solution comprising a spacer-compound, wherein the spacer-compound is preferably covalently bound to the surface, optionally a washing-step is performed, and subsequently the microparticles with the bound spacer-compound are incubated with the solution comprising CD28-specific superagonistic mAb, wherein the mAb are bound to the spacer-compound covalently or non-covalently,
e) the thus obtained cD28-specific superagonistic mAb bearing microparticles are separated from the solution and optionally submitted to a washing-step.

## Revendications

1. Microparticules comprenant une structure porteuse, dans lesquelles exclusivement des anticorps monoclonaux (mab) superagonistiques spécifiques de CD28 ou un composé de mimétisme de ceux-ci sont liés à la structure porteuse.

2. Microparticules selon la revendication 1, dans lesquelles les mab sont liés directement et de préférence covalentement à la surface de la structure porteuse.

3. Microparticules selon la revendication 1, dans lesquelles les mab sont liés indirectement à la surface de la structure porteuse par l'intermédiaire d'un composé espaceur lié de préférence covalentement à la surface de la structure porteuse.

4. Microparticules selon la revendication 3, dans lesquelles le composé espaceur est choisi à partir du groupe comprenant «des polymères organiques, des peptides, des protéines, et des combinaisons de telles substances».

5. Microparticules selon une des revendications 1 à 4, dans lesquelles la surface de la structure porteuse est formée par un polymère organique, qui de préférence est choisi à partir du groupe comprenant «le polystyrène, le polyuréthane, le polyester, la polyvinyle pyridine, la polyvinyle amine, la polyéthylène imine, le chitosane, et des mélanges de tels polymères».

6. Microparticules selon la revendication 5, dans lesquelles le polymère organique comprend des groupes réactifs, qui est par exemple l'éther de glycidyle.

7. Microparticules selon la revendication 5 ou 6, dans lesquelles le polymère organique est activé en surface par traitement avec un réactif d'activation, qui est de préférence la chlorure de p-toluène sulfonyl.

8. Microparticules selon une des revendications 1 à 7, dans lesquelles le diamètre de la structure porteuse est dans la gamme de 0,1 µm à 100 µm, de préférence dans la gamme de 1 µm à 20 µm, en particulier dans la gamme de 1 µm à 10 µm.

9. Microparticules selon une des revendications 1 à 8, dans lesquelles la surface de la structure porteuse (mesurée au moyen du procédé BET) est 1 à 10 fois, de préférence 1 à 4 fois la surface géométrique, sous supposition d'une surface de sphère lisse.

10. Utilisation de microparticules selon une des revendications 1 à 9 pour la production de moyens pour la stimulation de cellules du sang, en particulier de lymphocytes T, de lymphocytes B, de granulocytes, de monocytes et/ou thrombocytes.

11. Utilisation selon la revendication 10 pour la production d'une combinaison pharmaceutique pour le traitement de maladies avec des nombres réduits de cellules du sang, en particulier des nombres réduits de lymphocytes T, ou de maladies immunopathologiques ou pour l'accroissement de la réaction immune lors de vaccinations, dans laquelle un échantillon sanguin est pris d'un patient, dans laquelle optionnellement les cellules du sang sont isolées de l'échantillon sanguin, dans laquelle les cellules du sang sont cultivées in vitro sous l'addition d'une dose physiologiquement efficace de microparticules, et dans laquelle optionnellement les cellules du sang ainsi obtenues sont galéniquement préparées pour l'injection ou l'infusion.

12. Utilisation selon la revendication 10 pour la production d'une combinaison pharmaceutique pour le traitement de maladies avec des nombres réduits de cellules du sang ou de maladies immunopathologiques ou pour l'accroissement de la réaction immune lors de vaccinations, dans laquelle les microparticules sont galéniquement préparées de préférence pour l'injection ou l'infusion.

13. Procédé pour la production de microparticules selon une des revendications 1 à 9, comprenant les étapes suivantes:
a) des microparticules avec une surface formée d'un ou de plusieurs polymères organiques sont produites;
b) optionnellement la surface est activée;
c) les microparticules ainsi obtenues sont incubées avec une solution comprenant des mab superagonistiques spécifiques de CD28, les mab de préférence étant liés covalentement à la surface, ou
c') les microparticules ainsi obtenues sont d'abord incubées avec une solution comprenant un composé espaceur, ce composé espaceur étant lié de préférence covalentement à la surface, optionnellement après une étape de lavage a lieu, et puis les microparticules sont incubées avec le composé espaceur lié avec une solution comprenant des mab superagonistiques spécifiques de CD28, les mab étant liés covalentement ou non-covalentement au composé espaceur, et
d) les microparticules ainsi obtenues et portant des mab superagonistiques spécifiques de CD28 sont séparées de la solution et optionnellement soumises à une étape de lavage.
